# EUROPÄISCHE PATENTANMELDUNG

(11) **EP 1 378 852 A1**
(43) Veröffentlichungstag der Anmeldung: **07.01.2004**
(21) Anmeldenummer: 02014704.7
(22) Anmeldetag: 03.07.2002
(51) Int. Cl.: G06F 19/00

(54) **Verfahren und System zur Unterstützung der Therapieplanung, insbesondere bei Vorliegen multipler Defizite**

(71) Anmelder: SIEMENS AKTIENGESELLSCHAFT, 80333 München (DE); Dr. Hein GmbH, 90429 Nürnberg (DE)
(72) Erfinder: Abraham-Fuchs, Klaus, 91058 Erlangen (DE); Eisermann, Uwe, 91052 Erlangen (DE); Richter, Niels, 95349 Thurnau (DE); Setz, Robert, 91126 Rednitzhembach (DE)
(74) Vertreter: Berg, Peter, Dipl.-Ing.

(57) **Zusammenfassung**

Die vorliegende Erfindung betrifft ein Verfahren sowie ein System zur Unterstützung der Therapieplanung in der Rehabilitation bei Vorliegen multipler Defizite sowie zur Therapieplanung chronischer Krankheiten bei Vorliegen weiterer krankheitsbedingter Einschränkungen. Bei dem Verfahren werden ein Fähigkeits- oder Krankheitsprofil eines Patienten sowie zumindest eine erste Datenbank (11, 11a) bereitgestellt, die mehrere Therapiemodule und/oder behandelbare Fähigkeiten und den Therapiemodulen bzw. behandelbaren Fähigkeiten zugeordnete Mindestvoraussetzungen für die Durchführung der jeweiligen Therapiemodule bzw. die Behandlung der jeweiligen behandelbaren Fähigkeiten enthält. Von einer Datenverarbeitungsstation (10) wird das Fähigkeits- bzw. Krankheitsprofil des Patienten durch Zugriff auf die erste Datenbank (11, 11a) automatisch mit den Mindestvoraussetzungen verglichen und auf Basis des Vergleichs ein oder mehrere geeignete Therapiemodule und/oder geeignete behandelbare Fähigkeiten für einen Benutzer kenntlich gemacht, bei denen die Mindestvoraussetzungen erfüllt sind. Das Verfahren sowie das zugehörige System verringern den Zeitaufwand bei der Therapieplanung von Patienten mit multiplen Defiziten oder weiteren krankheitsbedingten Einschränkungen erheblich.

## Beschreibung

Die vorliegende Erfindung betrifft ein Verfahren sowie ein System zur Unterstützung der Therapieplanung in der Rehabilitation bei Vorliegen multipler Defizite sowie zur Therapieplanung chronischer Krankheiten bei Vorliegen von durch andere Krankheiten vorgegebener Einschränkungen.

Als Folge schwerer Erkrankungen wie bspw. Schlaganfall, Herzinfarkt oder Alzheimer'sche Krankheit oder als Folge schwerer operativer Eingriffe wie bspw. dem Einsatz von Gelenkimplantaten oder der Durchführung einer Amputation treten bei der Mehrzahl der Patienten unterschiedliche Defizite in der körperlichen und geistigen Leistungsfähigkeit auf. Diese Defizite sind in der Regel die Folge der Schwächung oder des kompletten Ausfalls einer Gehirnregion oder eines Muskels. Auch Kombinationen hiervon treten häufig auf. So kann z. B. eine Gehirnregion geschädigt sein, die für die Steuerung eines Muskels oder mehrerer Muskeln in funktionalen Ketten zuständig ist. Als Folge davon degenerieren die betroffenen Muskeln, so dass sie nicht mehr richtig eingesetzt werden können. Solche geistigen oder körperlichen Einschränkungen werden in der medizinischen Fachsprache als Fähigkeitsdefizite bezeichnet, die in verschiedene Fähigkeitsbereiche eingeteilt werden können. So ist unterscheidet eine bekannte Klassifizierung bspw.:
- motorische Fähigkeiten wie Kraft, Ausdauer, Beweglichkeit, Gleichgewicht, Reaktion, Orientierung, Differenzierung, Umstellung, Sprachmotorik;
- intellektuelle/kognitive Fähigkeiten wie Aufmerksamkeit, Gedächtnis, Planung, Sprachverständnis, Wortfindung, Sehen;
- organisch/physische Fähigkeiten wie bspw. die Verringerung der Organleistung;
- soziale Fähigkeiten wie bspw. die Kommunikations- und Partizipationsfähigkeit;
- emotionale Fähigkeiten wie bspw. die Fähigkeit zur Entwicklung des Selbstwertgefühls.
Einige Fähigkeiten erfordern auch ein Zusammenspiel motorischer und kognitiver Funktionen. So setzt bspw. die Tätigkeit des Treppensteigens Kraft und Gleichgewicht als motorische Fähigkeiten sowie Aufmerksamkeit und räumliche Wahrnehmung als kognitive Fähigkeiten voraus.

Sehr häufig tritt bei einem Patienten nicht ein einziges Defizit in einer Fähigkeitskategorie auf, sondern eine Kombination mehrerer Defizite in mehr oder weniger schwerer Ausprägung. Ziel einer therapeutischen Maßnahme, die meist im Rahmen eines Rehabilitationsprozesses durchgeführt wird, ist die Wiederherstellung der Fähigkeiten bzw. die weitest mögliche Verminderung der vorliegenden Defizite. Zu Beginn der rehabilitativen Maßnahme werden dabei in der Regel alle Fähigkeitsdefizite des Patienten anhand bekannter Verfahren zur Messung, Beobachtung und Befragung erfasst und ihr Ausmaß dokumentiert. Dieser Erfassungsprozess wird auch als Staging des Patienten bezeichnet. Je nach eingesetztem Messverfahren ist das Ergebnis dieses Staging-Prozesses quantitativ, bspw. in Prozent der Sehfähigkeit oder durch Angabe des Grades der Beweglichkeit des Oberarmes, oder qualitativ, bspw. durch eine Einstufung der Fähigkeitseinschränkung in schwer, mittelgradig oder leicht. Ein Beispiel eines etablierten Messverfahrens für das Staging zahlreicher neurologischer, kognitiver und psychischer Fähigkeiten ist die sog. Wiener Testbatterie der Firma Schuhfried.

Das Resultat dieser Eingangsuntersuchung ist im Idealfall ein fachübergreifender Fähigkeitsbericht, der sich in Form eines Fähigkeitsprofils darstellen lässt. Ein Fähigkeitsprofil wird in diesem Kontext als eine Liste aller relevanten Fähigkeiten und eine Zuordnung des Grades der Einschränkung dieser Fähigkeiten bei diesem Patienten zum Erhebungszeitpunkt definiert.

Neben dem Begriff Fähigkeiten wird in der medizinischen Fachsprache auch der Begriff Fertigkeit benutzt. Unter einer Fertigkeit wird im Kontext einer medizinischen Rehabilitationsmaßnahme eine komplexe Handlung verstanden, die aber in sich geschlossen und gegenüber anderen Handlungen abgrenzbar ist. Für eine Fertigkeit wird das Zusammenspiel mehrerer Fähigkeiten benötigt. Insbesondere bezieht sich der Begriff Fertigkeit im Kontext einer Rehabilitation auf Aktivitäten des täglichen Lebens (ADL: Activities of Daily Living), die Grundvoraussetzung für ein unabhängiges, selbständiges Leben sind. Beispiele für derartige Fertigkeiten sind das Einnehmen von Nahrung, das Anziehen, das Waschen, das Duschen, das Treppensteigen usw.. Die Ausführung solcher Fertigkeiten wird auch in standardisierten Fragebögen erfasst und als ADL-Index quantifiziert. Obwohl im Rahmen einer Rehabilitation in direkter Weise Fähigkeiten trainiert werden, ist das eigentliche Ziel die Wiedererlangung von Fertigkeiten. Insofern sind im Kontext der nachfolgenden Beschreibung die Begriffe Fähigkeit und Fertigkeit meist gegenseitig austauschbar.

In der Regel liegen bei einem Patienten mehrere Fähigkeitsdefizite gleichzeitig vor, die verschiedenen Fähigkeitskategorien gemäß der vorangegangenen Klassifizierung zugehören können. Bei der Therapieplanung muss der Arzt bei Patienten mit derartigen multiplen Defiziten Rücksicht auf gegenseitige Abhängigkeiten in der Behandlung der einzelnen Fähigkeitsdefizite nehmen, auch wenn diese nicht in seinen Zuständigkeitsbereich fallen. Bisher sind hierfür Besprechungstermine, mündliche Absprachen sowie der Austausch von schriftlichen Unterlagen zwischen den beteiligten Organisationseinheiten des Krankenhauses bzw. der Rehaklinik, den betreuenden Ärzten und Therapeuten und zwischen unterschiedlichen Leistungserbringern einer integrierten Gesundheitsversorgung erforderlich, um die Therapieplanung bei Patienten mit multiplen Defiziten durchführen zu können. Dies macht die Therapieplanung für den einzelnen Arzt oder Therapeuten zeitaufwendig und birgt die Gefahr, einzelne Abhängigkeiten in der Therapieplanung zu übersehen.

Eine vergleichbare Problematik tritt bei der Therapieplanung chronischer Krankheiten, wie Diabetes, Asthma o.ä., auf. Auch hier wird die Therapieplanung für den Arzt bei Vorliegen von durch andere Krankheiten des Patienten bedingten weiteren gesundheitlichen und körperlichen Einschränkungen erschwert.

Ausgehend von dieser Situation besteht die Aufgabe der vorliegenden Erfindung darin, ein Verfahren sowie ein System zur Unterstützung der Therapieplanung bei Vorliegen multipler Defizite oder von durch andere Krankheiten des Patienten bedingten Einschränkungen anzugeben, das den Zeitaufwand für den Arzt oder Therapeuten bei der Therapieplanung vermindert.

Die Aufgabe wird mit dem Verfahren sowie dem System gemäß den Patentansprüchen 1 bzw. 18 gelöst. Vorteilhafte Ausgestaltungen des Verfahrens sowie des Systems sind Gegenstand der Unteransprüche oder lassen sich aus der nachfolgenden Beschreibung sowie den Ausführungsbeispielen entnehmen.

Bei einer ersten Alternative des vorliegenden Verfahrens werden zur Unterstützung der Therapieplanung bei Vorliegen multipler Defizite ein Fähigkeitsprofil eines Patienten sowieeine erste Datenbank bereitgestellt, die mehrere Therapiemodule und/oder behandelbare Fähigkeiten und den Therapiemodulen bzw. behandelbaren Fähigkeiten zugeordnete Mindestvoraussetzungen von Fähigkeiten für die Durchführung der jeweiligen Therapiemodule bzw. die Behandlung der jeweiligen behandelbaren Fähigkeiten enthält. Unter Therapiemodulen sind bei dieser Alternative einzelne Übungen zu verstehen, die der Patient zur Wiedererlangung einzelner Fähigkeiten durchführen muss. Ein Therapiemodul kann bspw. ein Ergometertraining oder eine Gleichgewichtsübung umfassen. Unter Fähigkeit kann im vorliegenden Zusammenhang auch eine Fertigkeit verstanden werden, wobei dann das Therapiemodul für das Training einer speziellen Fertigkeit ausgebildet sein kann. Das Fähigkeitsprofil kann hierbei von einer Datenbank abgerufen oder auf eine Aufforderung der Datenverarbeitungsstation hin auch direkt vom Benutzer eingegeben werden. Beim vorliegenden Verfahren werden von einer Datenverarbeitungsstation das Fähigkeitsprofil des Patienten durch Zugriff auf die erste Datenbank automatisch mit den Mindestvoraussetzungen verglichen und auf Basis des Vergleichs ein oder mehrere geeignete Therapiemodule und/oder geeignete behandelbare Fähigkeiten für einen Benutzer kenntlich gemacht, bei denen die Mindestvoraussetzungen erfüllt sind. Die den in der Datenbank enthaltenen Therapiemodulen bzw. behandelbaren Fähigkeiten zugeordneten Mindestvoraussetzungen umfassen zumindest eine oder mehrere weitere erforderliche Fähigkeiten sowie vorzugsweise den Grad, zu dem diese Fähigkeiten für die Durchführung des jeweiligen Therapiemoduls bzw. die Behandlung der jeweiligen behandelbaren Fähigkeit vorhanden sein müssen. Das Fähigkeitsprofil des Patienten wird dabei vorzugsweise durch eine zweite Datenbank bereitgestellt, von der es durch die Datenverarbeitungsstation abgerufen wird. Diese zweite.Datenbank kann auch eine elektronische Patientenakte des Patienten sein. Vorzugsweise sind in der ersten Datenbank nur Therapiemodule bzw. behandelbare Fähigkeiten enthalten, die dem Benutzer für die Behandlung des Patienten zur Verfügung stehen. So kann die erste Datenbank individuell an ihren Einsatzort, bspw. das jeweilige Krankenhaus oder die jeweilige Fachabteilung, und die dort vorhandenen Therapiemöglichkeiten angepasst sein.

Bei einer zweiten Alternative des vorliegenden Verfahrens werden zur Unterstützung der Therapieplanung chronischer Krankheiten bei Vorliegen weiterer durch andere Krankheiten vorgegebener Einschränkungen ein Krankheitsprofil eines Patienten, das gesundheitsrelevante und körperliche Eigenschaften des Patienten umfasst, sowie eine erste Datenbank bereitgestellt, die mehrere Therapiemodule für die Behandlung chronischer Krankheiten und den Therapiemodulen zugeordnete Mindestvoraussetzungen von gesundheitsrelevanten und körperlichen Eigenschaften für die Durchführung der jeweiligen Therapiemodule enthält. Unter Therapiemodulen sind bei dieser Alternative Handlungen zu verstehen, die der Patient zur Behandlung seiner chronischen Krankheit durchführen muss. Ein Therapiemodul kann bspw. ein einen Zeitplan für die Durchführung von Inhalationen oder die Aufbringung einer Salbe umfassen. Beim vorliegenden Verfahren werden von einer Datenverarbeitungsstation das Krankheisprofil des Patienten durch Zugriff auf die erste Datenbank automatisch mit den Mindestvoraussetzungen verglichen und auf Basis des Vergleichs ein oder mehrere geeignete Therapiemodule für einen Benutzer kenntlich gemacht, bei denen die Mindestvoraussetzungen erfüllt sind. Das Krankheitsprofil des Patienten wird dabei vorzugsweise durch eine zweite Datenbank bereitgestellt, von der es durch die Datenverarbeitungsstation abgerufen wird. Diese zweite Datenbank kann auch eine elektronische Patientenakte des Patienten sein. Vorzugsweise sind in der ersten Datenbank nur Therapiemodule enthalten, die dem Benutzer für die Behandlung des Patienten zur Verfügung stehen. So kann die erste Datenbank individuell an ihren Einsatzort, bspw. das jeweilige Krankenhaus oder die jeweilige Fachabteilung, und die dort vorhandenen Therapiemöglichkeiten angepasst sein.

Mit dem vorliegenden Verfahren und dem zugehörigen System wird dem Benutzer, insbesondere dem Arzt oder Therapeuten, ein computerbasiertes Hilfsmittel zur Verfügung gestellt, mit dem er mit verringertem Zeitaufwand eine sichere Therapieplanung bei Patienten mit multiplen Defiziten oder bei Vorliegen weiterer durch andere Krankheiten vorgegebener Einschränkungen durchführen kann. Durch das Verfahren bzw. das zugehörige System wird das Fähigkeits- oder Krankheitsprofil des Patienten automatisch ausgewertet und durch Rückgriff auf eine oder mehrere Wissensbasen in Form einer oder mehrerer Datenbanken die geeigneten Therapiemodule bzw. behandelbaren Fähigkeiten kenntlich gemacht, die den gegenseitigen Abhängigkeiten bei der Behandlung multipler Defizite oder bei Vorliegen weiterer krankheitsbedingter Einschränkungen genügen. Der Benutzer kann anschließend die kenntlich gemachten behandelbaren Fähigkeiten bzw. Therapiemodule auswählen und zu einem Therapieplan zusammenstellen. Er muss hierbei nicht mehr eventuelle gegenseitige Abhängigkeiten berücksichtigen, da dies bereits automatisiert durch die Datenverarbeitungsstation erfolgt ist.

Im Folgenden wird die vorliegende Erfindung hinsichtlich der ersten Alternative des Verfahrens näher erläutert. Diese Erläuterungen lassen sich jedoch ohne Weiteres auch auf die zweite Alternaitve des Verfahrens anwenden, wenn das Fähigkeits- durch das Krankheitsprofil ersetzt wird, wobei sich dann die Mindestvoraussetzungen auf gesundheitsrelevante und körperliche Eigenschaften des Patienten beziehen.

Selbstverständlich muss das Fähigkeitsprofil bzw. Fertigkeitsprofil des Patienten bei der Durchführung des vorliegenden Verfahrens aktuell sein, um die einzelnen Abhängigkeiten sowie die zu behandelnden Fähigkeiten korrekt erkennen zu können. Die erste Datenbank kann bei einer Alternative des vorliegenden Verfahrens als erste Wissensbasis lediglich die Therapiemodule mit den zugeordneten Mindestvoraussetzungen enthalten. Die Mindestvoraussetzungen beinhalten dabei alle für die Durchführung des jeweiligen Therapiemoduls relevanten Fähigkeiten sowie den Grad der Fähigkeit, der für die Durchführung des Therapiemoduls erforderlich ist. Eine derartige Mindestvoraussetzung stellt bspw. die Bedingung dar, dass Fähigkeit X zumindest zu Y% vorhanden sein muss, wenn das entsprechende Therapiemodul verordnet werden soll. Alternativ oder ergänzend zu dieser ersten Wissensbasis kann eine zweite Wissensbasis in der ersten Datenbank implementiert oder als weitere Datenbank bereitgestellt werden, die eine Matrix von behandelbaren Fähigkeiten und/oder Fertigkeiten sowie eine Zuordnung von Mindestvoraussetzungen anderer Fähigkeiten bzw. Fertigkeiten F1 ... FN bei der Therapie der Fähigkeit FX enthält. So kann bspw. eine Mindestvoraussetzung lauten, dass die Fähigkeiten F1 ... FN zu zumindest Y% vorhanden sein müssen, um die Fähigkeit X behandeln zu können. Vorzugsweise enthält die erste und zweite Wissensbasis lediglich die dem jeweiligen Benutzer zur Verfügung stehenden Therapiemodule bzw. durch ihn behandelbaren Fähigkeiten. Auf diese Weise werden nur Therapiemodule bzw. Fähigkeiten ausgegeben, die in die Zuständigkeit des jeweiligen Arztes fallen.

Die Zuordnung der quantifizierten Abhängigkeiten in den beiden Wissensbasen erfolgt im Wesentlichen aus erworbener Erfahrung von Fachleuten auf diesem Gebiet bei der Behandlung von Patienten. In unterschiedlichen Ausführungen des vorliegenden Verfahrens sowie des zugehörigen Systems kann dieses Wissen bspw. durch Befragung eines oder mehrerer Experten gewonnen und in der ersten Datenbank abgelegt werden. In einer weiteren Ausführungsform wird die jeweilige Wissensbasis erst bei der Installation in einer Organisation gemeinsam mit dem künftigen Endnutzer gefüllt. Dieses Vorgehen hat den Vorteil, dass der Benutzer seine spezifischen Erfahrungen und die Randbedingung seiner Organisation bei der Erstellung der Wissensbasis berücksichtigen kann. Die Datenbanken enthalten unabhängig davon allgemein gültiges Wissen, das von der individuellen Situation eines Patienten unabhängig ist.

Die Kenntlichmachung der ein oder mehreren geeigneten Therapiemodule und/oder geeigneten behandelbaren Fähigkeiten erfolgt vorzugsweise dadurch, dass von der Datenverarbeitungsstation ausschließlich die Therapiemodule oder behandelbaren Fähigkeiten ausgegeben werden, bspw. durch Anzeige an einem Monitor, bei denen die Mindestvoraussetzungen erfüllt sind. In einer weiteren Ausgestaltung kann die Kenntlichmachung dadurch erfolgen, dass die Therapiemodule und/oder behandelbaren Fähigkeiten in einer Ausgabe einer größeren Anzahl von Therapiemodulen und/oder behandelbaren Fähigkeiten gekennzeichnet, bspw. graphisch hervorgehoben werden, bei denen die Mindestvoraussetzungen erfüllt sind.

Weiterhin können die geeigneten Therapiemodule bzw. geeigneten behandelbaren Fähigkeiten für einen Zugriff des Benutzers freigeschaltet und/oder die entsprechend nicht die Mindestvoraussetzungen erfüllende Therapiemodule bzw. behandelbaren Fähigkeiten für einen Zugriff des Benutzers bei der rechnergestützten Therapieplanung gesperrt werden. Selbstverständlich ist diese Sperrung oder Freischaltung eines Moduls oder einer Fähigkeit nur als entscheidungsunterstützender Hinweis des Systems zu verstehen. Der Benutzer, insbesondere der Arzt oder Therapeut, hat jederzeit die Möglichkeit, die Sperrung oder Freischaltung manuell zu verändern, wenn ihm dies aufgrund seiner Erfahrung angebracht erscheint. Bei manueller Änderung besteht die Option, in einem Eingabefeld eine Begründung für diese Änderung anzugeben, die abgespeichert wird.

Als Zusatz zur Sperrkennzeichnung kann der Grund angegeben sein, der zur Sperrung geführt hat, bspw. das Fähigkeitsdefizit, das als Ausschlusskriterium für die Nutzung des Therapiemoduls betrachtet wird. Der besseren Übersichtlichkeit halber kann diese Detailinformation auch erst dann erscheinen, wenn der Benutzer diese, bspw. durch Mausklick auf das gesperrte Modul, anfordert (Link auf die Ursache der Sperrung).

Vorzugsweise geben das vorliegende Verfahren sowie das zugehörige System nur die behandelbaren Fähigkeiten aus, die mit zu behandelnden Fähigkeiten übereinstimmen, die auf Basis des Fähigkeitsprofils von der Datenverarbeitungsstation bzw. einem darin enthaltenen Programmmodul ermittelt werden. Dies betrifft alle Fertigkeiten bzw. Fähigkeiten des Fähigkeitsprofils, bei denen Defizite vorliegen.

In einer weiteren Ausgestaltung des vorliegenden Verfahrens bzw. Systems wird eine dritte Datenbank bereitgestellt, die eine Zuordnung von Ziel-Fähigkeiten zu den Therapiemodulen enthält. Unter Ziel-Fähigkeiten werden die Fähigkeiten verstanden, die mit dem entsprechenden Modul trainiert werden. Die Datenverarbeitungsstation ist hierbei so ausgebildet, dass sie die Zuordnung zusammen mit den Therapiemodulen ausgibt und/oder nur die Therapiemodule ausgibt, bei denen die Ziel-Fähigkeiten mit einer oder mehreren vorher von einem Benutzer eingegebenen oder auf Basis des Fähigkeitsprofils von der Datenverarbeitungsstation automatisch ermittelten zu behandelnden Fähigkeiten übereinstimmen.

In einer weiteren Ausführungsform ist eine vierte Datenbank vorgesehen, die ebenso wie die dritte Datenbank auch Teil der ersten Datenbank sein kann. In dieser vierten Datenbank ist eine Zuordnung weiterer Mindestvoraussetzungen, die insbesondere gesundheitsrelevante und körperliche Eigenschaften des Patienten betreffen, für die Durchführung der Therapiemodule und/oder die Behandlung der behandelbaren Fähigkeiten enthalten. Diese weiteren Mindestvoraussetzungen umfassen Anforderungen und Ausschlusskriterien für jedes Therapiemodul über durch multiple Defizite bedingte Einschränkungen hinaus. So kann diese Datenbank bspw. Informationen darüber enthalten, dass bei Durchführung des Therapiemoduls X, z. B. Herzinsuffizienz, Osteoporose, Schwangerschaft, ein Herzschrittmacher, ein Gelenkimplantat, die Parkinson' sche Krankheit usw. nicht vorliegen darf. Weiterhin kann bspw. angegeben sein, dass für die Durchführung des Therapiemoduls X weitere Kriterien erfüllt sein müssen, bspw. dass der Patient ein vorgegebenes Höchstalter nicht überschreiten und ein vorgegebenes Mindestalter nicht unterschreiten darf, dass sein Körpergewicht innerhalb eines bestimmten Rahmens liegen muss oder dass der Patient ein bestimmtes Geschlecht haben muss. Diese weiteren Mindestvoraussetzungen werden ebenso automatisch von der Datenverarbeitungsstation bzw. dem darin enthaltenen Modul überprüft und bei dem Vergleich berücksichtigt. Die zugeordneten Patientendaten können dabei von einer weiteren Datenbank oder einer elektronischen Patientenakte abgerufen werden. Weiterhin kann die nötige Patienteninformation auch interaktiv vom Benutzer am Computerarbeitsplatz abgefragt werden oder durch Zugriff auf ein Krankenhausinformationssystem (Hospital Information System: HIS) beschafft werden. Durch diese Prüfung der weiteren Mindestvoraussetzungen werden beim vorliegenden Verfahren entsprechend wie bei der Prüfung der Mindestvoraussetzungen der Fähigkeiten ein oder mehrere Therapiemodule freigegeben bzw. bei Nichterfüllung gesperrt und ggf. wiederum mit einem entsprechenden Hinweis auf die Ursache der Sperrung versehen.

Bei dem vorliegenden Verfahren wird vorzugsweise im Verlauf einer Therapie mehrfach ein aktuelles Fähigkeitsprofil des Patienten abgerufen und automatisch mit den Mindestvoraussetzungen verglichen. Ergeben sich bei einem derartigen Vergleich andere Ergebnisse als bei dem vorangehenden Vergleich, so wird automatisch eine Meldung bzw. ein Hinweis an den Benutzer, insbesondere den zuständigen Therapeuten oder Arzt, generiert, um diesen auf die Veränderung hinzuweisen. Gleichzeitig werden betroffene Therapiemodule entsprechend der Erfüllung oder Nichterfüllung der Mindestvoraussetzungen freigeschaltet oder gesperrt. Auf diese Weise erhält der Benutzer jederzeit Informationen über eine Änderung der für die Therapieplanung wichtigen Entscheidungsgrundlagen, auch wenn diese auf Trainingsergebnissen beruhen, die nicht in seinen Zuständigkeitsbereich fallen. So kann bspw. durch ein Training, das der Patient in einer anderen Organisationseinheit bei einem Arzt-Kollegen durchführt, zum Erreichen eines Grades einer Fähigkeit führen, durch die die Mindestvoraussetzungen für die Durchführung eines bestimmten Therapiemoduls nunmehr vorliegen, das zu Beginn der Therapie mangels Erfüllung der Mindestvoraussetzungen gesperrt war. Dies wird dem Benutzer durch den generierten Hinweis oder eine besondere Kennzeichnung dieses Therapiemoduls in der Ausgabe kenntlich gemacht, der nun den Therapieplan entsprechend durch Einbeziehung des neu verfügbaren Therapiemoduls anpassen kann.

Eine derartige wiederholte Durchführung des Verfahrens im Verlauf der Therapie kann in regelmäßigen Zeitabständen oder auch nur dann erfolgen, wenn sich eine Veränderung in dem Fähigkeitsprofil des Patienten ergibt. Hierbei wird regelmäßig das Fähigkeitsprofil überprüft. Der Hinweis auf die aus der Veränderung resultierende Neubewertung der Therapiemodule oder behandelbaren Fähigkeiten kann bspw. durch eine E-mail, ein Fax o. ä. an den zuständigen Arzt oder Therapeuten erfolgen.

In einer weiteren Ausgestaltung des vorliegenden Verfahrens sowie des zugehörigen Systems enthält die erste Datenbank nicht nur die Mindestvoraussetzungen an Fähigkeiten für die Durchführung der jeweiligen Therapiemodule sondern auch die Voraussetzungen für die Durchführung einzelner Schwierigkeits- bzw. Belastungsstufen in Therapiemodulen. Die bereits beschriebenen Funktionen zur Sperrung, Freischaltung und Benachrichtigung werden dann nicht nur für abgeschlossene Therapiemodule benutzt, sondern auch für die entsprechenden Stufen innerhalb der Therapiemodule. Dann kann in analoger Weise durch Überprüfung des Fähigkeitsprofils des Patienten automatisch eine Schwierigkeitsstufe gesperrt oder freigegeben werden und dies dem Benutzer, bspw. an einem Monitor, dargestellt oder automatisch eine Meldung an diesen versandt werden.

Das zugehörige System zur Unterstützung der Therapieplanung umfasst entsprechend eine Datenverarbeitungsstation die mit der ersten Datenbank verbunden ist und ein Modul zum automatischen Vergleich eines vorgebbaren Fähigkeits- bzw. Krankheitsprofils mit den in der Datenbank enthaltenen Mindestvoraussetzungen durch Rückgriff auf die erste Datenbank und zur Kenntlichmachung ein oder mehrerer geeigneter Therapiemodule und/oder geeigneter behandelbarer Fähigkeiten auf Basis des Vergleichs, bei denen die Mindestvoraussetzungen erfüllt sind.

In den weiteren Ausgestaltungen des Systems ist die Datenverarbeitungsstation weiterhin mit der zweiten, dritten und vierten Datenbank verbunden, die bereits im Zusammenhang mit dem Verfahren erläutert wurden. Das Modul ist dabei jeweils zur Durchführung der im Zusammenhang mit dem Verfahren erläuterten automatisierten Verfahrensschritte ausgebildet.

Das vorliegende Verfahren sowie das zugehörige System werden nachfolgend anhand eines Ausführungsbeispiels in Verbindung mit den Zeichnungen ohne Beschränkung des allgemeinen Erfindungsgedankens nochmals kurz erläutert. Hierbei zeigen:
- Fig. 1: ein Beispiel für ein Fähigkeitsprofil eines Patienten (auszugsweise);
- Fig. 2: ein Beispiel für die Zuordnung von Therapiemodulen zu Mindestvoraussetzungen von Fähigkeiten in der ersten Datenbank;
- Fig. 3: ein Beispiel für eine Status-Darstellung der Therapiemodule, in der geeignete Therapiemodule kenntlich gemacht sind; und
- Fig. 4: einen Überblick über das vorliegende Verfahren sowie das zugehörige System in einer Ausgestaltung der Erfindung.

Im vorliegenden Ausführungsbeispiel wird der Vorgang der Therapieplanung durch einen Arzt oder Therapeuten unter Einsatz des vorliegenden Verfahrens sowie des zugehörigen Systems in einer speziellen Ausgestaltung beispielhaft erläutert. Das beispielhafte System umfasst einen Computerarbeitsplatz (Datenverarbeitungsstation 10) zur Therapieplanung und Therapieverlaufskontrolle mit einem Modul 16 zur automatisierten Auswertung der Daten. Die Datenverarbeitungsstation 10 ist mit verschiedenen Datenbanken verbunden, aus der das Modul 16 die erforderlichen Informationen abruft. In der Grundausstattung dieses beispielhaften Systems sind eine Datenbank 12 mit einem individuellen Fähigkeitsprofil des Patienten, eine erste Wissensdatenbank 11 mit Therapiemodulen und einer Zuordnung von Fähigkeits-Mindestvoraussetzungen, eine zweite Wissensdatenbank 11a mit behandelbaren Fähigkeiten und einer Zuordnung von Mindestvoraussetzungen anderer Fähigkeiten bzw. Fertigkeiten zur Therapie dieser Fähigkeit sowie eine dritte Datenbank 13 mit einer Zuordnung von Therapiemodulen zu mit dem jeweiligen Therapiemodul therapierten Ziel-Fähigkeiten vorgesehen.

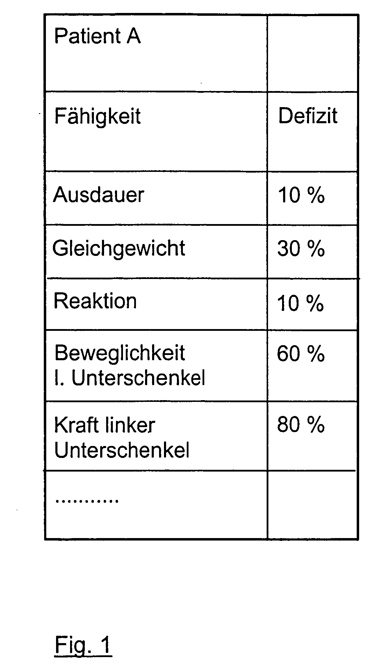

Ein Beispiel für ein Fähigkeitsprofil, wie es in der zweiten Datenbank 12 enthalten ist, zeigt Figur 1 im Auszug. Dieses Fähigkeitsprofil umfasst unterschiedliche Fähigkeiten, wie Ausdauer, Gleichgewicht usw. mit dem jeweiligen Defizit, d. h. dem Prozentsatz, zu dem bei diesem Patienten die jeweilige Fähigkeit gegenüber der 100%igen Fähigkeit einer gesunden Vergleichsperson verringert ist.

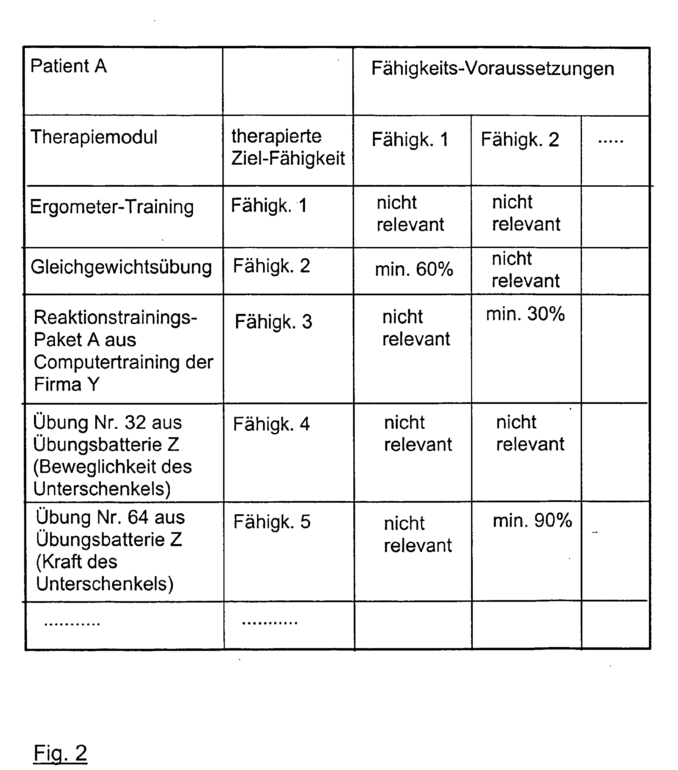

Die Wissens-Datenbank 11 umfasst alle dem Benutzer in seiner Organisationseinheit, bspw. dem Krankenhaus oder der Fachabteilung, zur Verfügung stehenden Therapiemodule sowie eine Zuordnung von Mindestvoraussetzungen aller für die Durchführung des jeweiligen Therapiemoduls relevanten Fähigkeiten. Der Inhalt dieser Datenbank ist beispielhaft in den Spalten 1 und 3 ff. der Figur 2 dargestellt. Die einzelnen Fähigkeiten sind hierbei in der vorliegenden Darstellung der Einfachheit halber lediglich durchnummeriert, in der Datenbank jedoch konkret angegeben oder der Nummerierung zugeordnet. Dieser Wissens-Datenbank 11 kann bspw. entnommen werden, dass für die Durchführung des Therapiemoduls "Reaktionstrainingspaket A aus Computertraining der Firma Y" die Fähigkeit 1 nicht relevant ist, die Fähigkeit 2 jedoch zu mindestens 30% vorhanden sein muss. Dieses Therapiemodul kann daher nur dann verordnet werden, wenn der Patient die Fähigkeit 2 zu mindestens 30% beherrscht. In gleicher Weise ist die zweite Wissens-Datenbank 11a ausgebildet, bei der lediglich an Stelle der Therapiemodule die entsprechenden behandelbaren Fähigkeiten stehen, so bspw. an Stelle des "Reaktionstrainingspakets A aus Computertraining der Firma Y" die "Fähigkeit 3". Diese zweite Wissens-Datenbank 11a kann als getrennte Datenbank ausgeführt sein oder Teil der ersten Wissens-Datenbank 1 sein.

Die vierte Datenbank 14, die ebenfalls Teil der Wissens-Datenbank 11 sein kann, umfasst eine Liste der zur Verfügung stehenden Therapiemodule sowie eine Zuordnung der Ziel-Fähigkeit bzw. Ziel-Fähigkeiten, die durch das jeweilige Therapiemodul behandelt werden können. Der Inhalt dieser Datenbank 14 entspricht somit den ersteh beiden Spalten der Figur 2.

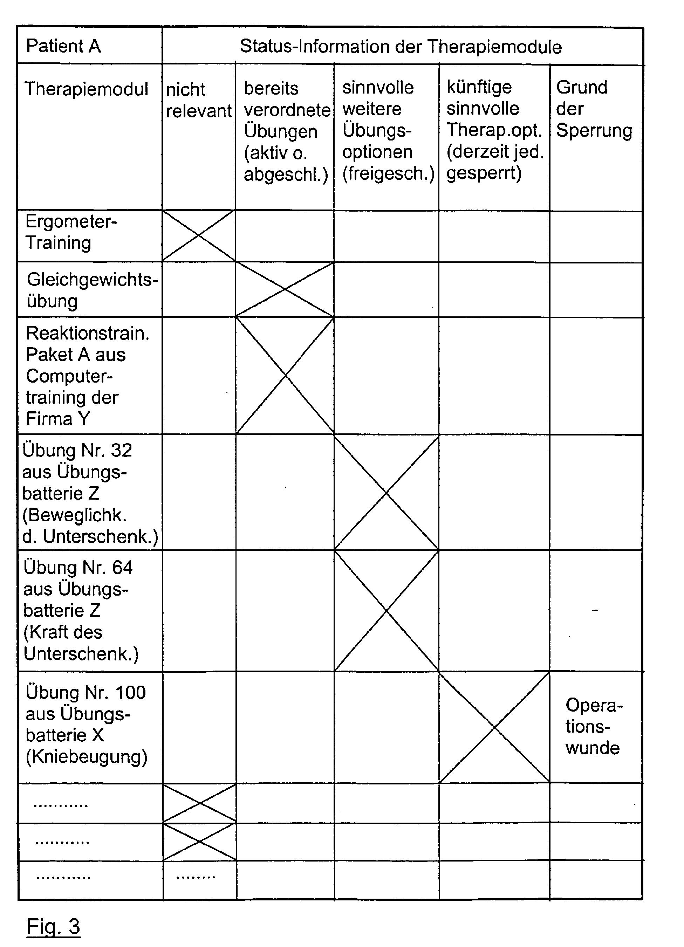

Bei der Benutzung des vorliegenden Verfahrens bzw. des zugehörigen Systems hat der Arzt oder Therapeut bei der Therapieplanung am Computerarbeitsplatz 10 Zugriff auf eine Liste aller Therapiemodule, die ihm zur Verordnung zur Verfügung stehen. Durch eine automatische Auswertung der Patientendatenbank (zweite Datenbank 12), der Wissens-Datenbanken 11, 11a und der vierten Datenbank 14 werden dem Arzt oder Therapeuten im vorliegenden Beispiel an einem Monitor des Computerarbeitsplatzes 10 alle zur Verfügung stehenden Therapiemodule aufgelistet und mit einer Statusinformation versehen, aus der ersichtlich ist, welche dieser Therapiemodule er dem Patienten auf Basis von dessen Fähigkeitsprofil verordnen kann. In dieser vollständigen und übersichtlichen Status-Darstellung des Therapiebedarfs und der möglichen Therapieoptionen, wie sie in Figur 3 beispielhaft angezeigt ist, kann der Arzt oder Therapeut ohne großen Zeitaufwand eine geeignete Therapieplanung vornehmen. Die Therapiemodule, für die der Patient die entsprechenden Mindestvoraussetzungen erfüllt, sind gekennzeichnet bzw. freigeschaltet. Therapiemodule, bei denen der Patient die Mindestvoraussetzungen an Fähigkeiten nicht erfüllt sind als gesperrt gekennzeichnet. Als Zusatz zur Sperrkennzeichnung ist im vorliegenden Fall auch der Grund der Sperrung angegeben. Somit hat der Planende eine sofortige Übersicht über die tatsächlich zur Verfügung stehenden Therapieoptionen, die ggf. zusätzlich graphisch hervorgehoben sein können. Auch die Art der Darstellung, wie sie in der Figur 3 nur zur Verdeutlichung wiedergegeben ist, kann graphisch wesentlich übersichtlicher gestaltet sein.

Im vorliegenden Beispiel sind zusätzlich die Therapiemodule als nicht relevant gekennzeichnet oder vollständig ausgeblendet, die bei dem Patienten nicht behandlungsbedürftig sind. Dies sind insbesondere Therapiemodule mit Ziel-Fähigkeiten, über die der Patient bereits zu 100% oder annähernd 100% verfügt. Weiterhin kann der Arzt oder Therapeut auch durch eine entsprechende Eingabe zu therapierende Fähigkeiten vorgeben. Neben den zum Zeitpunkt der Erfassung des Fähigkeitsprofils sinnvollen und einsetzbaren bzw. freigeschalteten Therapiemodulen sind im Beispiel der Figur 3 auch bereits in der Vergangenheit verordnete Module, die noch aktiv oder bereits abgeschlossen sein können, angegeben. Auch alle künftig sinnvollen, aber aufgrund bestehender multipler Defizite heute noch nicht einsetzbaren Therapiemodule können in der Darstellung entsprechend gekennzeichnet und gesperrt sein.

Selbstverständlich lässt sich durch den Arzt oder Therapeuten die Sperrung oder Freischaltung eines Therapiemoduls manuell verändern, wenn ihm dies aufgrund seiner Erfahrung angebracht erscheint. Für die weitere Therapieplanung wählt er dann die entsprechend freigeschalteten Therapiemodule oder eine Unterauswahl davon aus und stellt diese zu einem Therapieplan für den Patienten zusammen. Das System ist dabei vorzugsweise so ausgestaltet, dass sich die gesperrten Therapiemodule nicht in eine derartige Planung am Computerarbeitsplatz übernehmen lassen. Selbstverständlich gilt das Gleiche auch bei einer Planung unter Zugrundelegung der behandelbaren Fähigkeiten anstelle der Therapiemodule.

In einer weiteren Ausgestaltung des beispielhaften Systems steht eine dritte Wissens-Datenbank 3 zur Verfügung, die wiederum die zur Verfügung stehenden Therapiemodule und eine Zuordnung weiterer Anforderungen und Ausschlusskriterien für jedes Therapiemodul über durch multiple Defizite bedingte Einschränkungen hinaus umfasst. Diese weiteren Mindestvoraussetzungen können, wie bereits weiter oben angeführt, bestimmte Krankheiten oder Implantate wie auch grundsätzliche Patientendaten, wie das Alter, das Geschlecht oder das Gewicht sein. Zur Überprüfung des Vorliegens der entsprechenden zusätzlichen Mindestvoraussetzungen beim Patienten steht auch eine weitere Datenbank 12a, bspw. in Form einer elektronischen Patientenakte, zur Verfügung, die diese weiteren Daten enthält. Auch in diesem Fall kann selbstverständlich die Datenbank 11 mit dem Fähigkeitsprofil mit der Datenbank 11a mit den weiteren Patientendaten zusammengefasst sein.

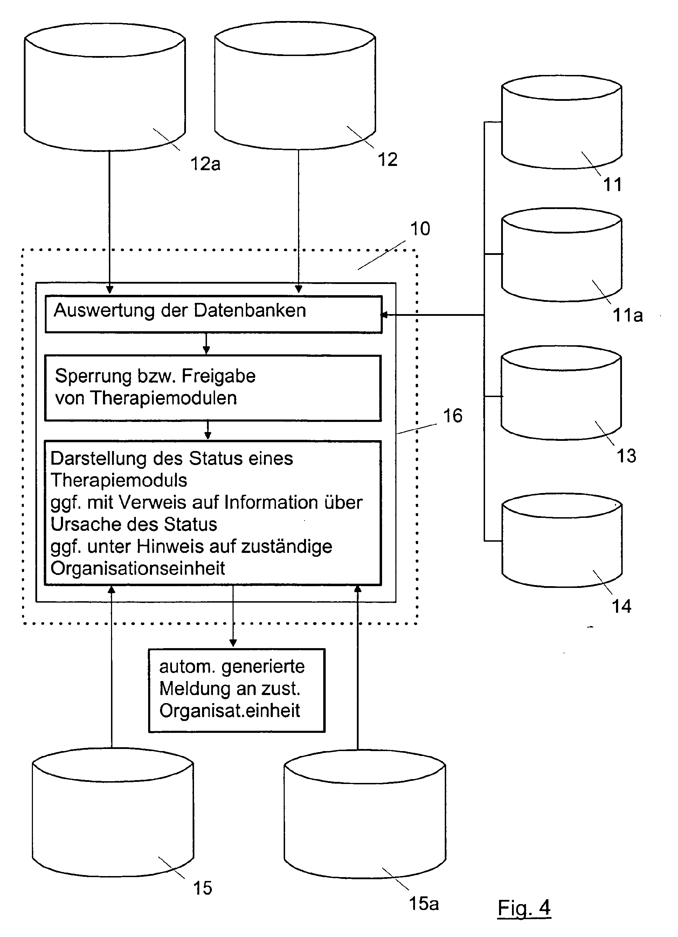

Die entsprechende Kennzeichnung sowie Sperrung bzw. Freigabe von Therapiemodulen durch das Modul 16 der Datenverarbeitungsstation 11 erfolgt durch Auswertung aller relevanten Datenbanken, wie dies in der Figur 4 schematisch dargestellt ist. Die entsprechenden Therapiemodule werden mit ihrem Status bzw. ihrer Kennzeichnung vorzugsweise an einem Monitor dargestellt. Es versteht sich von selbst, dass die Darstellung auch in anderer Form, bspw. durch Ausgabe auf einem Drucker, erfolgen kann. In der Figur 4 ist eine weitere Ausgestaltung des vorliegenden Systems sowie des vorliegenden Verfahrens angedeutet, bei dem die Datenverarbeitungsstation 10 Zugriff auf weitere Datenbanken 15, 15a hat, in denen einzelnen Fähigkeiten bzw. Therapiemodulen Organisationseinheiten, ggf. gegliedert nach Organisationskategorien, zugeordnet sind. Mit Hilfe dieser weiteren Datenbanken 15, 15a hat der planende Therapeut oder Arzt zusätzlich noch die Übersicht, für welche relevanten Therapiemodule andere Organisationseinheiten, bspw. andere Ärzte oder Fachabteilungen, zuständig sind. Insbesondere sieht er im Verlauf der Therapieerfolgskontrolle und bei der Anpassung der Therapie an den Therapieverlauf, welche Defizite gleichzeitig von anderen Fachabteilungen behandelt werden, die derzeit Ausschlusskriterien für ein ansonsten angebrachtes Therapiemodul in seinem Verantwortungsbereich darstellen. Bei einer entsprechenden Verbesserung dieser Defizite über einen kritischen Schwellwert hinaus, der der Mindestvoraussetzung des ansonsten angebrachten Therapiemoduls entspricht, wird in der ausgegebenen Übersicht sofort das bisher gesperrte Therapiemodul freigeschalten. Dies kann durch ein besonderes Hinweis-Flag gekennzeichnet werden. Weiterhin kann automatisch eine Meldung an die für das nunmehr freigeschaltete Therapiemodul zuständige Organisationseinheit, insbesondere den dort zuständigen Arzt oder Therapeuten, generiert und übermittelt werden.

Darüber hinaus kann das System bei Veränderung der Datenbank der Fähigkeitsprofile des Patienten oder bei Veränderung in der elektronischen Patientenakte des Patienten automatisch prüfen, ob ein relevanter Schwellwert eines Defizits - eine Mindestvoraussetzung - des Patienten, das zur Sperrung oder Freischaltung eines Therapiemoduls führt, durch die aktuelle Veränderung des Patientenzustandes über- oder unterschritten wird. Auch in diesem Fall kann an die jeweils zuständige Organisationseinheit bzw. den zuständigen Arzt oder Therapeuten eine Meldung, bspw. per E-mail oder Fax, versendet werden, so dass dieser den Hinweis auf eine notwendige Veränderung im-Falle der Sperrung eines Therapiemoduls oder eine angebrachte Veränderung im Falle der Freischaltung eines Therapiemoduls sofort erfährt, auch wenn er den Therapieplanungs-Arbeitsplatz gerade nicht benutzt. Dies erfordert eine wiederholte Überprüfung des Fähigkeits-Profils des Patienten in der zweiten Datenbank 12 bzw. der elektronischen Patientenakte 12a durch die Datenverarbeitungsstation 10.

Durch das beispielhaft dargestellte Verfahren und zugehörige System werden durch Nutzung von Datenbanken und graphischen Bedienungsoberflächen an einem Computerarbeitsplatz dem behandelnden Arzt oder Therapeuten automatisch Informationen bereitgestellt, die für die Zulässigkeit der Verordnung eines Therapiemoduls im Arbeitsablauf der Therapieplanung und Therapieverlaufskontrolle zur Behandlung von Fähigkeitsdefiziten relevant sind. Der Planende erhält eine sofortige Übersicht über die unter Berücksichtigung der multiplen Defizite und ggf. weiterer Einschränkungen tatsächlich zur Verfügung stehenden Therapieoptionen für die Therapieplanung.

## Patentansprüche

1. Verfahren zur Unterstützung der Therapieplanung bei Vorliegen multipler Defizite, bei dem ein Fähigkeitsprofil eines Patienten sowie eine erste Datenbank (11, 11a) bereitgestellt werden, die mehrere Therapiemodule und/oder behandelbare Fähigkeiten und den Therapiemodulen bzw. behandelbaren Fähigkeiten zugeordnete Mindestvoraussetzungen von Fähigkeiten für die Durchführung der jeweiligen Therapiemodule bzw. die Behandlung der jeweiligen behandelbaren Fähigkeiten enthält, und von einer Datenverarbeitungsstation (10) das Fähigkeitsprofil des Patienten durch Zugriff auf die erste Datenbank (11, 11a) automatisch mit den Mindestvoraussetzungen verglichen und auf Basis des Vergleichs ein oder mehrere geeignete Therapiemodule und/oder geeignete behandelbare Fähigkeiten für einen Benutzer kenntlich gemacht werden, bei denen die Mindestvoraussetzungen erfüllt sind.

2. Verfahren zur Unterstützung der Therapieplanung chronischer Krankheiten bei Vorliegen weiterer durch andere Krankheiten vorgegebener Einschränkungen, bei dem ein Krankheitsprofil eines Patienten, das gesundheitsrelevante und körperliche Eigenschaften des Patienten umfasst, sowie eine erste Datenbank (11) bereitgestellt werden, die mehrere Therapiemodule zur Behandlung von chronischen Krankheiten und den Therapiemodulen zugeordnete Mindestvoraussetzungen von gesundheitsrelevanten und körperlichen Eigenschaften für die Durchführung der jeweiligen Therapiemodule enthält, und von einer Datenverarbeitungsstation (10) das Krankheitsprofil des Patienten durch Zugriff auf die erste Datenbank (11) automatisch mit den Mindestvoraussetzungen verglichen und auf Basis des Vergleichs ein oder mehrere geeignete Therapiemodule für einen Benutzer kenntlich gemacht werden, bei denen die Mindestvoraussetzungen erfüllt sind.

3. Verfahren nach Anspruch 1 oder 2,
**dadurch gekennzeichnet,**
**dass** die Kenntlichmachung der ein oder mehreren geeigneten Therapiemodule und/oder geeigneten behandelbaren Fähigkeiten dadurch erfolgt, dass ausschließlich die geeigneten Therapiemodule und/oder die geeigneten behandelbaren Fähigkeiten ausgegeben, insbesondere an einem Monitor angezeigt, werden.

4. Verfahren nach Anspruch 1 oder 2,
**dadurch gekennzeichnet,**
**dass** die Kenntlichmachung der ein oder mehreren geeigneten Therapiemodule und/oder geeigneten behandelbaren Fähigkeiten dadurch erfolgt, dass die geeigneten Therapiemodule und/oder geeigneten behandelbaren Fähigkeiten in der Ausgäbe, insbesondere in einer Anzeige an einem Monitor, gekennzeichnet werden.

5. Verfahren nach einem der Ansprüche 1 bis 4,
**dadurch gekennzeichnet,**
**dass** das Fähigkeits- oder Krankheitsprofil des Patienten durch die Datenverarbeitungsstation (10) von einer zweiten Datenbank (12) abgerufen wird.

6. Verfahren nach einem der Ansprüche 1 bis 5,
**dadurch gekennzeichnet,**
**dass** die geeigneten Therapiemodule bzw. geeigneten behandelbaren Fähigkeiten für einen Zugriff freigeschaltet und/oder verbleibende Therapiemodule bzw. verbleibende behandelbare Fähigkeiten, bei denen die Mindestvoraussetzungen nicht erfüllt sind, für einen Zugriff gesperrt werden.

7. Verfahren nach Anspruch 6,
**dadurch gekennzeichnet,**
**dass** die gesperrten Therapiemodule bzw. Fähigkeiten in der Ausgabe mit einem Hinweis über den Grund der Sperrung versehen werden.

8. Verfahren nach Anspruch 6,
**dadurch gekennzeichnet,**
**dass** zu den gesperrten Therapiemodulen bzw. Fähigkeiten auf eine entsprechende Eingabe des Benutzers hin ein Hinweis über den Grund der Sperrung ausgegeben wird.

9. Verfahren nach einem der Ansprüche 6 bis 8,
**dadurch gekennzeichnet,**
**dass** die gesperrten Therapiemodule bzw. Fähigkeiten durch eine manuelle Eingabe eines Benutzers einzeln freigeschaltet werden können.

10. Verfahren nach einem der Ansprüche 1, 3 bis 9,
**dadurch gekennzeichnet,**
**dass** die Datenverarbeitungsstation (10) nur die geeigneten behandelbaren Fähigkeiten ausgibt, die mit zu behandelnden Fähigkeiten übereinstimmen, die auf Basis des Fähigkeitsprofils von der Datenverarbeitungsstation (10) ermittelt werden.

11. Verfahren nach einem der Ansprüche 1, 3 bis 9,
**dadurch gekennzeichnet,**
**dass** eine dritte Datenbank (13), gegebenenfalls als Teil der ersten Datenbank (11, 11a) bereitgestellt wird, die eine Zuordnung von Ziel-Fähigkeiten zu den Therapiemodulen enthält, wobei die Datenverarbeitungsstation die Zuordnung zusammen mit den Therapiemodulen ausgibt und/oder nur die geeigneten Therapiemodule ausgibt, bei denen die Ziel-Fähigkeiten miteiner oder mehreren vorher eingegebenen oder auf Basis des Fähigkeitsprofils von der Datenverarbeitungsstation (10) ermittelten zu behandelnden Fähigkeiten übereinstimmen.

12. Verfahren nach einem der Ansprüche 1 bis 11,
**dadurch gekennzeichnet,**
**dass** die Datenverarbeitungsstation (10) die Therapiemodule mit einer Statusanzeige ausgibt, die eine Information über eine bereits durchgeführte Verordnung der Therapiemodule und/oder eine zukünftige Eignung der Therapiemodule nach Vorliegen bestimmter Voraussetzungen umfasst.

13. Verfahren nach einem der Ansprüche 1, 3 bis 12,
**dadurch gekennzeichnet,**
**dass** weitere Patientendaten sowie eine vierte Datenbank (14), gegebenenfalls als Teil der ersten Datenbank (11, 11a), bereitgestellt werden, die eine Zuordnung weiterer Mindestvoraussetzungen für die Durchführung der Therapiemodule und/oder die Behandlung der behandelbaren Fähigkeiten enthält, und die Datenverarbeitungsstation (10) die weiteren Mindestvoraussetzungen bei dem Vergleich berücksichtigt.

14. Verfahren nach einem der Ansprüche 1 bis 12,
**dadurch gekennzeichnet,**
**dass** im Verlauf einer Therapie mehrfach ein aktuelles Fähigkeits- oder Krankheitsprofil des Patienten durch die Datenverarbeitungsstation (10) abgerufen und automatisch mit den Mindestvoraussetzungen verglichen wird, um bei einer Änderung des Fähigkeits- bzw. Krankheitsprofils, die sich auf die Erfüllung der Mindestvoraussetzungen einzelner Therapiemodule und/oder behandelbarer Fähigkeiten auswirkt, automatisch einen Hinweis an den Benutzer zu generieren und gegebenenfalls die betroffenen Therapiemodule bzw. Fähigkeiten zu sperren oder freizuschalten.

15. Verfahren nach Anspruch 14,
**dadurch gekennzeichnet,**
**dass** der Abruf des aktuellen Fähigkeits- oder Krankheitsprofils in vorgebbaren Zeitintervallen oder auf eine bei Änderung des Fähigkeits- bzw. Krankheitsprofils automatisch generierte Nachricht hin erfolgt.

16. Verfahren nach Anspruch 14 oder 15,
**dadurch gekennzeichnet,**
**dass** der Hinweis an den Benutzer von der Datenverarbeitungsstation (10) über ein elektronisches Medium, insbesondere per Email oder Fax, versendet wird.

17. Verfahren nach einem der Ansprüche 1 bis 16,
**dadurch gekennzeichnet,**
**dass** durch die Datenverarbeitungsstation (10) einzelne Teilstufen innerhalb der Therapiemodule in Abhängigkeit von der Erfüllung der Mindestvoraussetzungen freigeschaltet oder gesperrt werden.

18. System zur Unterstützung der Therapieplanung in der Rehabilitation eines Patienten, mit einer Datenverarbeitungsstation (10), die mit einer ersten Datenbank (11, 11a) verbunden ist, die mehrere Therapiemodule und/oder behandelbare Fähigkeiten und den Therapiemodulen bzw. behandelbaren Fähigkeiten zugeordnete Mindestvoraussetzungen von Fähigkeiten für die Durchführung der jeweiligen Therapiemodule bzw. die Behandlung der jeweiligen behandelbaren Fähigkeiten enthält, und ein Modul (16) zum automatischen Vergleich eines vorgebbaren Fähigkeitsprofils mit den Mindestvoraussetzungen durch Rückgriff auf die erste Datenbank (11, 11a) und zur Kenntlichmachung ein oder mehrerer geeigneter Therapiemodule und/oder geeigneter behandelbarer Fähigkeiten auf Basis des Vergleichs, bei denen die Mindestvoraussetzungen erfüllt sind.

19. System zur Unterstützung der Therapieplanung chronischer Krankheiten eines Patienten, mit einer Datenverarbeitungsstation (10), die mit einer ersten Datenbank (11) verbunden ist, die mehrere Therapiemodule zur Behandlung von chronischen Krankheiten und den Therapiemodulen zugeordnete Mindestvoraussetzungen von gesundheitsrelevanten und körperlichen Eigenschaften für die Durchführung der jeweiligen Therapiemodule enthält, und ein Modul (16) zum automatischen Vergleich eines vorgebbaren Krankheitsprofils, das gesundheitsrelevante und körperliche Eigenschaften des Patienten umfasst, mit den Mindestvoraussetzungen durch Rückgriff auf die erste Datenbank (11) und zur Kenntlichmachung ein oder mehrerer geeigneter Therapiemodule auf Basis des Vergleichs, bei denen die Mindestvoraussetzungen erfüllt sind.

20. System nach Anspruch 18 oder 19,
**dadurch gekennzeichnet,**
**dass** das Modul (16) zur Ausgabe ausschließlich der ein oder mehreren geeigneten Therapiemodule und/oder geeigneten behandelbaren Fähigkeiten, insbesondere an einem Monitor, ausgebildet ist.

21. System nach Anspruch 18 oder 19,
**dadurch gekennzeichnet,**
**dass** das Modul (16) zur Ausgabe der Therapiemodule und/oder behandelbaren Fähigkeiten und zur Kennzeichnung der ein oder mehreren geeigneten Therapiemodule und/oder geeigneten behandelbaren Fähigkeiten in der Ausgabe, insbesondere an einem Monitor, ausgebildet ist.

22. System nach einem der Ansprüche 18 bis 21,
**dadurch gekennzeichnet,**
**dass** das Modul (16) zur Freischaltung der geeigneten Therapiemodule und/oder geeigneten behandelbaren Fähigkeiten für einen Zugriff und/oder zur Sperrung von verbleibenden Therapiemodulen und/oder behandelbaren Fähigkeiten für einen Zugriff, bei denen die Mindestvoraussetzungen nicht erfüllt sind, ausgebildet ist.

23. System nach einem der Ansprüche 18 bis 22,
**dadurch gekennzeichnet,**
**dass** die Datenverarbeitungsstation (10) mit einer zweiten Datenbank (12) verbunden ist, aus der das Fähigkeits- oder Krankheitsprofil abrufbar ist.

24. System nach einem der Ansprüche 18, 20 bis 23,
**dadurch gekennzeichnet,**
**dass** das Modul (16) zur Ausgabe ausschließlich der geeigneten behandelbaren Fähigkeiten ausgebildet ist, die mit zu behandelnden Fähigkeiten übereinstimmen, die auf Basis des Fähigkeitsprofils vom Modul ermittelt werden.

25. System nach einem der Ansprüche 18, 20 bis 24,
**dadurch gekennzeichnet,**
**dass** in der ersten Datenbank (11, 11a) oder einer dritten Datenbank (13), mit der die Datenverarbeitungsstation verbunden ist, eine Zuordnung von Ziel-Fähigkeiten zu den Therapiemodulen enthalten ist, und das Modul (16) zur Ausgabe der Zuordnung zusammen mit den Therapiemodülen und/oder zur Ausgabe ausschließlich der geeigneten Therapiemodule ausgebildet ist, bei denen die Ziel-Fähigkeiten mit einer oder mehreren vorher eingegebenen oder auf Basis des Fähigkeitsprofils vom Modul (16) ermittelten zu behandelnden Fähigkeiten übereinstimmen.

26. System nach einem der Ansprüche 18, 20 bis 25,
**dadurch gekennzeichnet,**
**dass** in der ersten Datenbank (11, 11a) oder einer vierten Datenbank (14), mit der die Datenverarbeitungsstation (10) verbunden ist, eine Zuordnung weiterer Mindestvoraussetzungen für die Durchführung der Therapiemodule und/oder die Behandlung der behandelbaren Fähigkeiten enthalten ist, und das Modul (16) zur Berücksichtigung der weiteren Mindestvoraussetzungen bei dem automatischen Vergleich ausgebildet ist.
